Europäisches Patentamt

(19) European Patent Office

Office européen des brevets

(11) Publication number: **0 267 998**
**A1**

## (12) EUROPEAN PATENT APPLICATION

(21) Application number: **86402548.1**

(22) Date of filing: **17.11.86**

(51) Int. Cl.⁴: **A61K 39/10**

Amended claims in accordance with Rule 86 (2) EPC.

(43) Date of publication of application:
**25.05.88 Bulletin 88/21**

(84) Designated Contracting States:
**AT BE CH DE ES FR GB GR IT LI LU NL SE**

(71) Applicant: **INSTITUT PASTEUR**
**25-28, rue du Docteur Roux**
**F-75724 Paris Cédex 15(FR)**

(72) Inventor: **Guiso-Maclouf, Nicole**
**13 bld Saint-Marcel**
**F-75013 Paris(FR)**
Inventor: **Brezin, Colette**
**11 bis, avenue E. Deschanel**
**F-75007 Paris(FR)**
Inventor: **Rocancourt, Murielle**
**26 rue Lazare Carnot**
**F-92140 Clamart(FR)**
Inventor: **Alonso, Jean-Michel**
**51 rue de Cornouailles**
**F-78180 Montigny Le Bretonneux(FR)**

(74) Representative: **Peaucelle, Chantal et al**
**S.C. Ernest Gutmann - Yves Plasseraud 67,**
**boulevard Haussmann**
**F-75008 Paris(FR)**

(54) **Means for protecting against bordetella infections and toxic processes.**

(57) The invention relates to vaccine compositions consisting in FHA in association with a pharmaceutical carrier.

EP 0 267 998 A1

## MEANS FOR PROTECTING AGAINST BORDETELLA INFECTIONS AND TOXIC PROCESSES

The invention relates to means for protecting against Bordetella infections and toxic processes.

It relates more particularly to the use of factors responsive for the adhesion of Bordetella on the host cellular receptors.

Amongst the virulence factors indentified in Bordetella pertussis, the whooping cough bacillus, two proteins have clearly demonstrated biological effects : the filamentous hemagglutinin (FHA), major adherence factor (1), and the pertussis toxin (Ptx), also known as leucocytosis-promoting factor or histamine-sensitizing factor (2) or islet-activating protein (3), according to its various biological effects. Ptx is an ADP-ribosylating toxin that alters the inhibitory regulation of eucaryotic adenylate cyclase (4). The FHA and Ptx are considered as efficient immunogens against whooping cough.

B. pertussis also produces an extracellular adenylate cyclase (AC) whose biological effects have been only tested in vitro by exposing mammalian cells to crude or purified preparations , and by demonstrating the dramatic increase of intracellular cyclic adenosine 3′-5′ monophosphate (cAMP). Indirect evidence for the role of AC in the lethal infection of the mouse has been also reported. But one of the major difficulties in testing the virulence and the immunogenicity of Bordetella pertussis is the lack of experimental model mimicking the infectious process of the disease, that involves colonization of the ciliated respiratory epithelium, followed by local alteration of the target cells and systemic effects due to the pertussis toxin, such as leucocytosis, hyperinsulinemia, or sensitization to histamine.

Experimental challenges are usually performed by using the intracerebral injection (5) or aerosol administration (6) to mice, of virulent inocula. However the intracerebral test only provides informations upon the ability of the mouse to overcome the lethal effects of the experimental infection, but do not allow quantitative measurement of the host-bacteria relationship during the course of the infectious process and the aerosol tests require special equipment.

The inventors, thanks to a murin model of respiratory infection they have elaborated, have been able to investigate the protective effects of antibodies to virulence factors of Bordetella, more particularly to FHA and also Ptx.

The invention is based on the discovery that antibodies anti-FHA are able to fully protect the host against the lethal infections caused by Bordetella .

It is then an object of the invention to provide means for protecting against Bordetella infections and toxic processes, more particularly efficient vaccine compositions.

The vaccine compositions of the invention are characterized in that they consist in FHA in association with a pharmaceutical carrier.

The experiments carried on by the inventors have shown indeed that administration of a FHA preparation to humans or animals is particularly efficient to prevent Bordetella infections and toxic processes and is devoid of toxic effects. On the contrary, it has been established that the use of Ptx, associated with PHA, gives side-effects. Particularly, it was shown that Ptx is an element which can induce encephalitis. Such risks are then eliminated by the invention since FHA is not associated with Ptx in the vaccine compositions.

In the vaccine compositions of the invention, FHA is either associated with AC in the same inoculum or not, under the proviso that AC does not give crossed reactions with the host AC.

Alternatively, FHA is administered at the same time than AC or at different times.

The use in association with AC enables both to prevent pathogen bacteria attachment and to neutralize against the main toxin, under the proviso mentioned above that AC does not react with the host eucaryote AC.

The administration is preferably carried out by the intranasal route or the parenteral route, using the usual pharmaceutical carriers, at the usual dosis.

FHA preparations are advantageously obtained using the method, for example, of Sato et al. in Infect Immun, 1983, 41, 313-320 or Imaizum et al., Journal of Microbiol. Methods, 2, 334-347 (1984).

Preferred AC preparations useful as vaccine compositions consist in the purified AC preparations disclosed in FR patent application, filed on November 17, 1986, in the name of the applicant, relating to "Purified AC preparations, a method for obtaining the same and their biological applications". Particularly preferred vaccine compositions with AC preparations are elaborated with the active epitope specifically recognized by a monoclonal antibody raised against said purified AC preparations. Such a monoclonal antibody is designated as 8-25 and is obtained from hybridoma strain I-910 whose strain was deposited at "Collection Nationale de Cultures de Microorganismes" (CNCM) on October 9, 1986.

Advantageously, the vaccine compositions of the invention prevent the infection caused by Bordetella, such as B. pertussis, B. parapertussis, B. bronchiseptica and B. cervicum.

Other characteristics and advantages of the reference to the figures, wherein :

-fig. 1A and 1B represent the kinetics of growth of B. pertussis in the lungs and of WBC counts respectively ;

-fig. 2A to 2C, micrographs of lesions in the lungs ;

-fig. 3A, the kinetics of growth of B. pertussis in the lungs, and fig. 3B, the WBC counts before and after iv injections of 0.5 µg of pertussis toxin ;

-fig. 4A and 4B, elicitation with 4 x 10⁸ CFU heated at 56°C and 80°C respectively.


## EXAMPLE

Experimental disease in mice and study of immune responses

### Material and methods

#### a) Infection of mice :

Three weeks old, specified pathogen-free, C57B1/6 and Balb/c female mice, weighing 16 g ± 1 were purchased from the ferme expérimentale de Rennemoulin (Institut Pasteur, 78 Villepreux, France) and maintained by groups of 5 in cages topped with filter caps (Tecniplast, UAR, France), on sterile litter, and fed with gamma-ray sterilized food (granulés RO3, UAR, France) and autoclaved drinking water. The mice were infected at 4 weeks of age by intranasal injection of standardized bacterial inocula (in a volume of 50 ul) from the B. pertussis strain Kendrick 18323 (type strain ATCC 9797), after short ether anesthesia.

#### b) Culture conditions and bacterial counts :

B. pertussis 18323, stored at -70°C, in Stainer Scholte modified medium (SS) supplemented with 30 % glycerol, was grown on Bordet Gengou agar (Diagnostic Pasteur, 92 Marnes la Coquette, France) supplemented with defibrinated horse blood at the final concentration of 15 % (BGB) for 72 h at 36°C ± 1. From this culture, one hemolytic colony was subcultured in the same conditions prior to suspension in sterile saline for extemporaneous injection to mice. Viable bacteria in the inocula or from the lungs of mice, sacrified, by cervical dislocation, homogenized in glass-tubes with teflon pestles (Tri-R Laboratory Instruments, Prosciences, Paris, France) were counted as colony-forming units (CFU) on BGB, sampled with ten-fold serial dilutions and incubated as indicated above (the trachea and the main bronchia could not be used for measurement of B. pertussis in vivo growth, because, in most instances, they were contaminated with streptococci or unidentified Bacillus sp., in low amounts, 10¹ to 10² per organ, but enough to overgrow the cultures and mask B. pertussis colonies).

#### c) Histopathological examinations :

The trachea and the lungs were fixed in 10 % formaldehyde for 1 week prior to embedding in paraffin, serial sectioning and staining with haematoxylin and eosin or Gram or Giemsa for light microscopy.

#### d) Leucocyte counts :

White blood cells (WBC) were counted in a haemocytometer from blood samples collected from the retroorbital plexus of mice punctured, at fixed time each day, with a single use pipetting system (Unopette, Bekton-Dickinson, Rutherford, New Jersey, USA).

#### e) Tests for acquired immunity :

Anti-pertussis toxin and anti-filamentous hemagglutinin antibodies in convalescing mice were tested by using an Enzyme-Linked Immunosorbent Assay (ELISA), described in (7). Anti-B. pertussis whole cell agglutinins were tested in U-bottomed microplates using as antigen either a suspension of B. pertussis 18323 in saline, heat-inactivated at 56°C, or a commercial preparation (Bordetella pertussis antigen, Difco Laboratories, Detroit Michigan, USA).

Delayed-type hypersensitivity reactions were tested, as described in (7), by following the local inflammatory reaction in the footpads of mice injected with B. pertussis 24 h culture in SS, (4 x 10⁸ CFU in 40 µl), heated at 56°C or at 80°C for 20 minutes.

Acquired resistance to reinfection was tested in mice that had recovered from a respiratory tract infection, induced with 1.4 x 10⁵ CFU, 30 days before, and in control non immune mice, aged 8 weeks, by intranasal challenge with 1.2 x 10⁶ CFU.


## RESULTS

1-Dose-effects-of intranasal challenge of C57B1/6 and Balb/c mice with B.pertussis18323 :

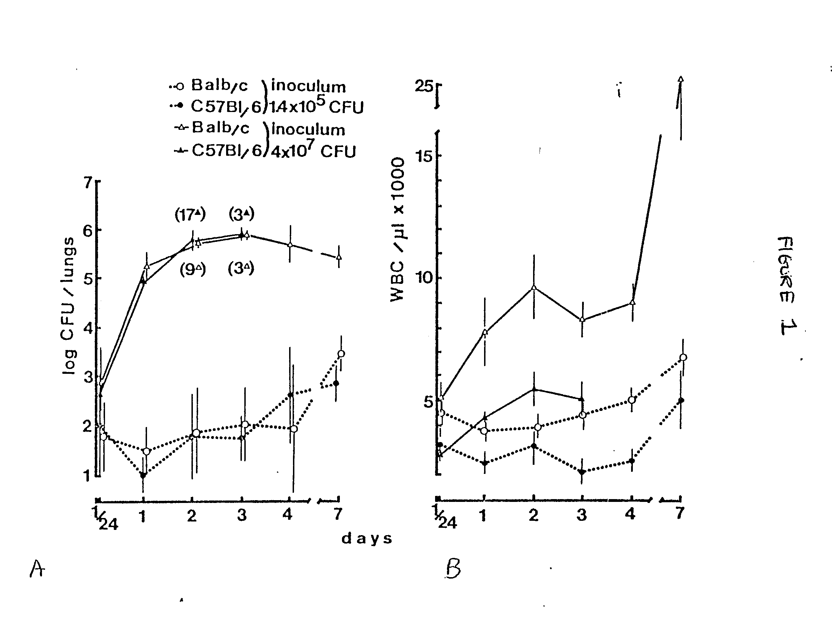

The dose-effects of the intranasal infection are reported on figures 1A and 1B which respectively give in function of days the variations of log CFU/lungs 1B, and of WBC counts/μl x 1000 (the vertical bars are arythmetic means of 5 mice ± SEM).

As shown in figure 1A, the amount of CFU recovered from the lungs of mice of both strains evolved in parallel, according to the inoculum. At low dose, the bacterial counts were highly variable among the mice of both strains, contrasting with the narrow variance of data obtained with the high dose. At high dose, the inoculum reaching the lungs was only about 10 fold higher than with the 285 fold lower dose, but the amount of CFU recovered from the lungs increased more than 100 fold within 24h, reaching, at 48h, values of about $10^6$ CFU, that correlated with the emergence of lethal effects. It appears from figure 1B that the WBC increase depended upon the intensity of the infection.

## 2-Histopathology :

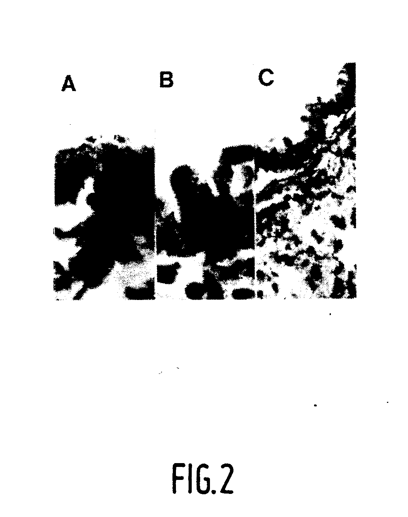

The histopathological lesions in the lungs of mice infected intranasally with B.pertussis were studied with respect to the adherence of B.pertussis to the cilia of the epithelial cells (section of bronchiole, Giemsa staining, x 1000), (figure 2A), the turgidity of shed epithelial cells (section of a bronchiole, Giemsa staining, x 1000), (figure 2B), and the turgid aspect of the epithelium in a bronchiole and associated congestive a veolitis (lung section, Giemsa staining, x 4000), (figure 2C).

Light microscopy examination of sections of the trachea and lungs of mice of both strains, at day 2 post intranasal injection of 8 x $10^6$ CFU, evidence similar patterns (figure 2) : few clusters of bacteria were only found associated with the ciliated epithelium of the trachea of the bronchia, mostly in the bronchioles were the epithelial cells had a turgescent aspect ; these pictures were associated with congestion of the alveolar capillaries, producing sometimes aspects of oedematous alveolitis.

## 3-Acquired immunity :

Acquired resistance to reinfection with B. pertussis and to the leucocytose promoting effects of the pertussis toxin was studied in C57B1/6 and Balb/c mice, convalescent or not from a respiratory infection with B. pertussis.

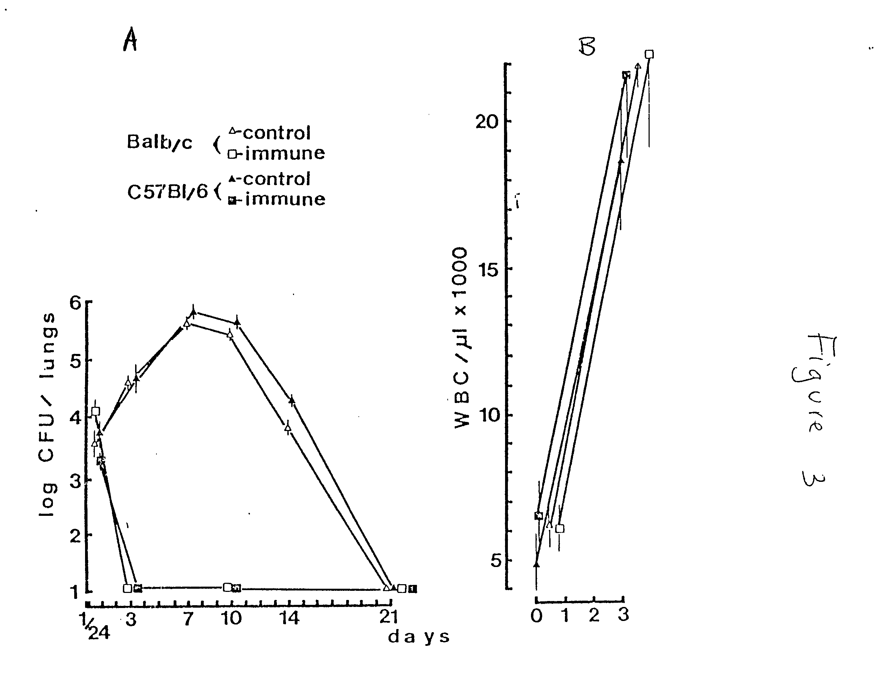

On figure 3, part A are reported the kinetics of growth of B. pertussis in the lungs, and on part B WBC counts before and after iv injection of 0.5 μg of pertussis toxin (the vertical bars are arythmetic means of 5 mice ± SEM).

It appears that C57B1/6 mice and Balb/c mice, convalescing from a respiratory infection with B.pertussis , became resistant to reinfection. As shown in figure 3, though the amount of CFU recovered from the lungs, 1h after intranasal infection, was not significantly different among convalescing mice and controls, the bacteria were cleared from the lungs of convalescing mice in less than 3 days, whereas a bacterial proliferation, reaching a maximum at day 7 and then decreasing until day 21, was observed in control mice. In this experiment a 2.5 fold increase in WBC counts was reached at day 14, decreasing to normal values at day 21, in control C57B1/6 or Balb/c mice, whereas no significant leucocytosis was detected in convalescing mice. However, these results could not be interpreted as the expression of an acquired resistance to the effects of the leucocytosis-promoting factor of the pertussis toxin in convalescing mice, since no bacterial proliferation was detected in these hosts (the release of pertussis toxin from the cleared incoculum was probably too low to induce detectable leucocytosis).

In fact, acquired resistance to the leucocytosis promoting effects of the infection with B.pertussis was tested by injecting iv 0.5 μg of purified pertussis toxin to convalescing as well as to control mice and by measuring th WBC increase between day 0 and 3. As shown in figure 3, both groups of mice underwent equivalent WBC increase.

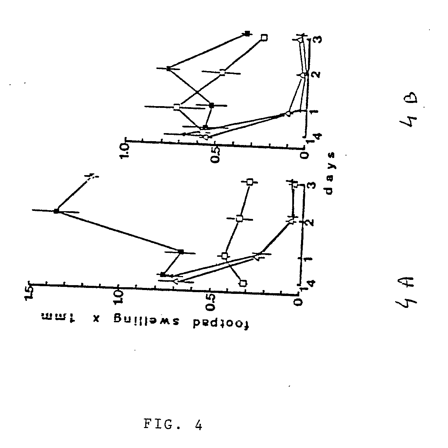

Sera collected from the mice before and at day 21 after the challenge presented in figure 3, did not allow to detect antibodies to the filamentous haemagglutinin or the pertussis toxin, by ELISA (starting dilution of 1:20). Agglutinins to B.pertussis whole cells antigens were only detected in Balb/c mice, 21 days after reinfection, at the lower dilution of 1:20. The only significant immune response to B.pertussis detected in convalescing mice, was a delayed-type hypersensitivity (DTH) reaction to whole cell antigens as shown on figures 4A and 4B. Figure 4A represents elicitation with 4 x $10^8$ CFU heated at 56°C and figure B, elicitation with 4 x $10^8$ CFU heated at 80°C (the symbols are the same than in figure 3).

## 4-Protective effects of anti-FHA B.pertussis - (antibodies against lethal challenges) :

The specific inhibitory effects of antibodies raised against whole cell antigens, FHA, Ptx, was tested in a series of experiments.

The results obtained are given in the following table :

Table :Protective effects of various anti-B.pertussis antibodies against lethal challenges.

| | Controls | Normal serum | Anti-whole cell vaccine | | Anti-FHA | | | Anti-Ptx | | Anti-AC (polyclonal) | | | Anti-AC (monoclonal) | | | |
|---|---|---|---|---|---|---|---|---|---|---|---|---|---|---|---|---|
| $\log_{10}$ serum dilution | | -1 | -1 | -2 | -1 | -1.6 | -2 | -1 | -2 | -1 | -1.6 | -2.2 | -2.2 | -2.5 | -2.8 | -3.1 |
| Exp.1:1.6x10$^9$CFU/ml[*] | | | | | | | | | | | | | | | | |
| (AC=76 u/ml)[+] | | | | | | | | | | | | | | | | |
| Lethality[‡] | 14/15 | 4/5 | 4/5 | 4/5 | 0/5 | 0/5 | 3 | 4/5 | 5/5 | 0/5 | 0/5 | 3/5 | | | | |
| % AC inactivation[§] | 0 | 0 | 0 | 0 | 0 | 0 | 0 | 0 | 0 | 89 | 84.5 | 56 | | | | |
| % Ptx inhibition[o] | 0 | 0 | 0 | 0 | 0 | 0 | 0 | 100 | 100 | 0 | 0 | 0 | | | | |

[*]density of the inoculum

[+]AC activity in the inoculum supernatant (1 unit of AC corresponds to 1 umol of cAMP formed in 1 min at 30°C at pH 8)

[‡]n° dead mice/total

[§]in vitro inactivation of the extracellular AC in the inoculum

[o]inhibition of CHO-clustering effects of the inoculum supernatant

As shown in the above table, the lethal respiratory challenge with a virulent clone of B. pertussis was highly reproducible, it induced the death of the mice within 4 days. Survivors at day 5 fully recovered.

By preincubating the challenge inoculum with various sera, it was found that only anti-FHA and anti-AC antibodies were protective against the acute haemorrhagic alveolitis in non immunized controls.

The anti-Ptx antibodies, though having protective effects against cytopathic effects on cultured cells, failed to protect the mice against the experimental challenge.

The protective effects of anti-FHA were probably due to the efficient inhibition of bacterial adherence by these antibodies, leading to rapid clearance of B.pertussis from the surface of the target epithelium.

The articles to which it is referred in the specification are given hereinafter :

1. Tuomanen E. & Weis A., J. Infect. Dis., 152, 118-125 (1985)

2. Morse S. I. & Morse J. H., J. Exp. Med., 143, 483-502 (1976)

3. Yajima M. K., Hosoda K., Kanbayashi Y., Nakamura Y., Takahashi L. & Ui M., J. Biochem. (Tokyo), 83, 305-312 (1978)

4. Hsia J. A., Moss J., Hewlett E. L. & Vaughan M., J. Biol. Chem., 259, 1086-1090 (1984)

5. Medical Research Council Whooping Cough Immunisation Committee (1956), Br. Med. J., 2, 454-462

6. Sato Y., Izumiya H., Sato H., Cowell J. L. & Manclark C. R., Infect. Immun., 29, 261-266 (1980)

7. Alonso J. M., Megret F., Bresin C., Friedman R. L. & Alouf J. E., FEMS Microbiol. Lett. (in press) (1986)

## Claims

1. Vaccine composition characterized in that it consists in FHA in association with a pharmaceutical carrier.

Amended claims in accordance with Rule 86(2) EPC.

1. Vaccine compositions characterized in that they consist in FHA in association with a pharmaceutical carrier.

2. Vaccine compositions according to claim 1, wherein FHA is either associated with adenylate cyclase or AC in the same inoculum or not, under the proviso that AC does not give crossed reactions with the host AC.

3. Vaccine compositions according to claim 1, wherein FHA is administered at the same time than AC or at different times.

4. Vaccine compositions according to claim 2 or 3, wherein the AC preparations are elaborated with the active epitope specifically recognized by a monoclonal antibody raised against purified AC preparations.

5. Vaccine compositions according to claim 4, wherein the monoclonal antibody is 8-25 subclone, obtained from hybridoma strain I-610 deposited at the "Collection Nationale de Cultures de Microorganismes" (CNCM) on October 9, 1986.

6. Vaccine compositions according to anyone of claims 1 to 5 for preventing the infection caused by Bordetella which are administered by the intranasal or the parenteral route.

0 267 998

**FIGURE 1**

o Balb/c ⎫ inoculum
• C57Bl/6 ⎭ 1.4×10$^5$ CFU
△ Balb/c ⎫ inoculum
▲ C57Bl/6 ⎭ 4×10$^7$ CFU

log CFU / lungs

(17▲)  (3▲)
(9▲)  (3▲)

$\frac{1}{24}$  1  2  3  4  7

days

A

WBC /µl ×1000

25
15
10
5

$\frac{1}{24}$  1  2  3  4  7

B

FIG.2

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 175 841 (JURIDICAL FOUNDATION THE CHEMO-SERO-THERAPEUTIC RESEARCH INSTITUTE) * page 1, lines 4-18 * | 1 | A 61 K 39/10 |
| X | EP-A-0 159 003 (JURIDICAL FOUNDATION THE CHEMO-SERO-THERAPEUTIC RESEARCH INSTITUTE) * abstract; page 3, lines 24-28; page 4, lines 1-4 * | 1 | |
| X | PATENT ABSTRACTS OF JAPAN, vol. 10, no. 107 (C-341)[2164], 22nd April 1986; & JP - A - 60 237 027 (KAGAKU OYOBI K.K.) 25-11-1985 * whole document * | 1 | |
| X | EP-A-0 162 639 (THE WELLCOME CORP.) * page 2, lines 3-16; claims * | 1 | TECHNICAL FIELDS SEARCHED (Int. Cl.4) A 61 K 39/00 A 61 K 37/00 |

The present search report has been drawn up for all claims

| Place of search | Date of completion of the search | Examiner |
|---|---|---|
| BERLIN | 06-05-1987 | AVEDIKIAN P.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82